Europäisches Patentamt

European Patent Office

Office européen des brevets

(19)

(11) Numéro de publication : **0 297 934 B1**

# (12) FASCICULE DE BREVET EUROPEEN

(45) Date de publication du fascicule du brevet :
16.10.91 Bulletin 91/42

(51) Int. Cl.⁵ : **G01T 1/164**

(21) Numéro de dépôt : **88401289.9**

(22) Date de dépôt : **26.05.88**

(54) **Procédé de prise en compte des impulsions de localisation délivrées par une gamma-caméra.**

(30) Priorité : **27.05.87 FR 8707479**

(43) Date de publication de la demande :
**04.01.89 Bulletin 89/01**

(45) Mention de la délivrance du brevet :
**16.10.91 Bulletin 91/42**

(84) Etats contractants désignés :
**DE GB NL**

(56) Documents cités :
**EP-A- 0 131 478**
**FR-A- 2 546 632**

(73) Titulaire : **COMMISSARIAT A L'ENERGIE ATOMIQUE Etablissement de Caractère Scientifique Technique et Industriel 31/33, rue de la Fédération F-75015 Paris (FR)**

(72) Inventeur : **Bonnefoy-Claudet, Jean-Paul 9, rue Alfred Fredet F-38100 Grenoble (FR)**
Inventeur : **Mestais, Corinne Le Prieuré Bernin F-38190 Brignoud (FR)**

(74) Mandataire : **Mongrédien, André et al c/o BREVATOME 25, rue de Ponthieu F-75008 Paris (FR)**

EP 0 297 934 B1

Jouve, 18, rue Saint-Denis, 75001 PARIS

## Description

La présente invention a pour objet un procédé de prise en compte des impulsions de localisation délivrées par une gamma-caméra. Elle trouve son application particulièrement dans le domaine médical où des gamma-caméras sont utilisées pour produire des images d'organes à examiner, en vue d'établir un diagnostic. Elle se rapporte aux caméras à scintillation, ou gamma-caméra, de type ANGER dont le brevet américain 3.011.057 décrit le fonctionnement dans ses principes et ses moyens de réalisation. Ces gamma-caméras sont destinées à détecter et à visualiser des photons émis par des corps radioactifs.

Les gamma-caméras sont utilisées en médecine nucléaire pour visualiser dans un organe la répartition de molécules marquées par un isotope radioactif que l'on a injecté à un patient. Une gamma-caméra comprend généralement un collimateur pour focaliser les photons gamma émis par le patient, un cristal scintillateur pour transformer les photons gamma en photons lumineux ou scintillations, et un réseau de tubes photomultiplicateurs qui transforment chacun les scintillations en des impulsions électriques dites contributions électriques de tubes. Une gamma-caméra comprend en outre des circuits électroniques pour produire, à partir des contributions électriques fournies par les tubes photomultiplicateurs, des signaux de coordonnées X et Y du lieu où s'est produite la scintillation, ainsi qu'un signal de validation Z quand l'amplitude de la scintillation appartient à une bande d'énergie prédéterminée.

Cette chaine de détection est suivie d'un ensemble de visualisation comportant généralement un oscilloscope cathodique commandé par les signaux de coordonnées X, Y, et par Z pour visualiser par un point lumineux sur l'écran le point d'impact du photon gamma sur le cristal. L'ensemble de visualisation peut éventuellement comporter un dispositif photographique pour former une image de l'organe observé en intégrant un grand nombre de points lumineux produits sur l'écran cathodique. Il peut par ailleurs comprendre un dispositif de traitement numérique des images. Notamment, le dispositif de traitement numérique des images peut être utilisé pour reconstruire des images de coupe des organes examinés afin de produire des tomographies de ces organes. Dans ce dernier cas des algorithmes de reconstruction d'images identiques à ceux utilisés en tomodensitométrie sont mis en oeuvre.

Entre autre qualités une gamma-caméra doit posséder une bonne résolution spatiale, c'est à dire la capacité de distinguer des petites sources radioactives rapprochées, une bonne réponse en taux de comptage, c'est à dire la capacité de traiter un grand nombre d'évènements par unité de temps, et une qualité d'image indépendante de l'énergie de l'isotope considéré. La résolution spatiale dépend de la précision du calcul des coordonnées X et Y. La qualité de l'élaboration de ces coordonnées dépend essentiellement des lois physiques régissant le fonctionnement des différentes parties de la gamma-caméra. Ainsi l'interaction d'un photon gamma avec le cristal donne naissance à une scintillation lumineuse dont l'intensité décroît exponentiellement avec le temps. La constante de temps de cette décroissance est caractéristique du cristal scintillateur utilisé. Pour un cristal d'iodure de sodium activé au thallium, Nal (Tl), elle est de l'ordre de 250 nanosecondes. Cette scintillation est vue par plusieurs tubes photomultiplicateurs simultanément. Les photons lumineux composant cette scintillation arrachent des photoélectrons aux photocathodes des tubes photomultiplicateurs. Le nombre de photoélectrons arrachés obéit, pour une scintillation donnée, à la loi statistique de Poisson. Ceci signifie que la contribution électrique d'un tube photomultiplicateur recevant une scintillation a une amplitude dont la valeur suit une distribution statistique de Poisson. La valeur moyenne de cette amplitude est fonction de l'énergie des photons lumineux incidents.

Comme une scintillation est vue par plusieurs tubes photomultiplicateurs simultanément, la détermination de l'emplacement de cette scintillation sur le cristal, elle-même représentative du lieu d'émission du photon gamma d'excitation est obtenue en calculant l'emplacement du barycentre des contributions électriques délivrées par l'ensemble des tubes photomultiplicateurs excités par cette scintillation. Ce calcul s'effectue simplement, selon ANGER, en injectant les contributions électriques au travers d'un jeu de matrices de résistances. Les valeurs des résistances des matrices sont fonction, pour des matrices dites de localisation, des positions des tubes photomultiplicateurs auxquels elles sont raccordées. Les positions de ces tubes sont repérées par rapport à des axes cartésiens de référence dont le point d'intersection est généralement situé au centre du réseau de tubes.

Le problème le plus difficile à résoudre consiste, pour une scintillation donnée, à déterminer le plus exactement possible la valeur moyenne des amplitudes de chacune des contributions. Aussi il est connu d'intégrer dans le temps ces contributions sur une période de l'ordre de trois fois la constante de temps de décroissance des scintillations du cristal scintillateur. La durée d'intégration, ou durée de comptage, dépend de la constante de temps du cristal. La précision de la mesure est entachée d'erreurs dûes à la fluctuation statistique de Poisson. En effet, l'écart type de la fluctuation de l'amplitude des contributions selon la statistique de Poisson est inversement proportionnelle à la racine carrée du nombre de photoélectrons arrachés. Ainsi, plus l'intégration est longue, plus le nombre de photoélectrons pris en compte est important, et aussi plus l'écart type est faible et donc plus la valeur moyenne de cette contribution est appréciée avec exactitude.

En fait, l'opération de calcul de l'emplacement du barycentre étant une opération linéaire, il est plus économique de réaliser cette intégration à la sortie de chacune des matrices de résistances du jeu de matrices. En effet, ces matrices n'effectuent qu'une pondération de ces contributions en fonction de l'emplacement des tubes sur le cristal. Les impulsions électriques délivrées en sortie du jeu des matrices de résistances sont dites impulsions pondérées. On notera au passage que la durée de comptage est directement liée à la qualité de la résolution spatiale de la gamma-caméra et que cette qualité s'obtient au détriment du taux de comptage, c'est à dire au détriment du nombre d'évènements par seconde pris en compte.

L'opération d'intégration ne va en fait pas sans quelques difficultés. Dans une précédente demande de brevet français n° 83.08824 déposée le 27 mai 1983 une telle gamma-caméra a été décrite. Elle comporte, en plus de tous les circuits électroniques servant à la localisation des scintillations, des circuits de validation pour prendre en compte des scintillations dont l'amplitude se situe dans une gamme prédéterminée, et qui ne sont pas suivies, après la durée d'intégration, par l'arrivée inopinée d'une autre scintillation, éventuellement même produite en un autre endroit sur le cristal, et dont l'intervention risque de fausser le calcul de la localisation. La détermination de la présence d'une scintillation y est faite en comparant l'amplitude d'un signal temporel, dit signal énergie, à un seuil constituant une des deux bornes de la gamme prédéterminée. En plus de la comparaison du signal énergie à un seuil, on étudie le passage par zéro de la dérivée par rapport au temps de ce signal. La différenciation ainsi entreprise marque, lors de ce passage à zéro, le point culminant d'une impulsion pondérée formant ce signal et représentative de l'énergie. Si cette crête se produit après le franchissement du seuil bas de la gamme, et si le seuil haut de la gamme n'est pas franchi, on en déduit que la scintillation est à prendre en compte.

Lorsqu' un phénomène dit d'empilement se produit, une deuxième scintillation naît avant que les effets de la première ne soient complètement évanouis. Dans la demande de brevet citée ci-dessus un circuit logique est prévu pour interdire la prise en compte de cette seconde scintillation si elle se produit au bout d'une durée trop faible après l'apparition de la première. Cependant rien n'est prévu pour rejeter la prise en compte de la scintillation qui vient d'être mesurée, si pendant la durée d'intégration de cette première scintillation une autre scintillation se produit. On connaît un dispositif qui permet d'arriver à ce résultat. Il consiste à utiliser un même circuit logique que celui qui sert à la détermination de la présence d'une scintillation, et ceci pendant la durée nécessaire à l'intégration de cette scintillation. Si le circuit logique rend compte de la présence d'une deuxième crête, on peut décider d'abandonner la mesure effectuée et de ne pas tenir compte de sa signification.

Cette dernière méthode présente cependant un inconvénient. En effet, elle ne donne pas de résultat cohérent dans la mesure où une scintillation est toujours suivie de parasites de scintillation qui créent pendant le temps de décroissance du signal des crêtes parasites dont la détection provoque le rejet de la mesure de la scintillation entreprise. En définitive si un tel circuit fonctionne parfaitement, compte tenu du bruit de scintillation, le taux de comptage de la gamma caméra devient nul : tous les coups sont rejetés parce qu'ils sont entachés de bruits. Si on veut avec le circuit logique prendre en compte une valeur de seuil on se heurte à une difficulté quasiment insurmontable du fait que le signal temporel de la scintillation décroît pendant la période d'intégration. Il devient alors difficile de comparer à un seuil donné, un signal résultant de la somme de la décroissance (attendue) du signal de scintillation et d'un signal parasite (aléatoire) à détecter. De ce qui précède, on a été conduit à négliger, dans l'état de la technique, l'apparition des empilements. Aussi quand ceux-ci se produisent ils viennent perturber le signal de localisation. Il importe donc, pour améliorer la résolution spatiale de l'image, de résoudre ce problème.

On pourra également se reporter à l'état de la technique constitué par le document EP-A-0131478. Cependant comme dans les autres dispositifs connus il n'y a pas de mesure réelle de l'énergie de scintillation.

La présente invention a pour objet de remédier à ces inconvénients en proposant un procédé de prise en compte dans lequel on ne se préoccupe pas de la valeur du signal temporel représentatif de l'énergie, sinon plus de la dérivée de ce signal, mais au contraire de son intégration pendant une durée prédéterminée. De préférence cette durée prédéterminée est égale à la durée d'intégration utilisée pour calculer les impulsions de localisation. Il devient alors possible de définir à l'issue de cette intégration une plage de la valeur de l'énergie de la scintillation dans laquelle on accepte de prendre en compte les impulsions de localisation calculées. Ainsi, par rapport à une fenêtre de spectrométrie caractéristique de l'isotope utilisé on peut définir une autre fenêtre de spectrométrie, dont le seuil bas est maintenu, et dont le seuil haut est déplacé positivement d'une certaine valeur de manière à tenir compte des scintillations entachées de bruit, et de manière à ne pas trop altérer le taux de comptage de la camera. En outre, en réglant la proportion admissible de dépassement de la fenêtre de spectrométrie par l'énergie de scintillation, on peut régler la tolérance d'empilement que l'on accepte. Aucun des systèmes connus ne comporte une telle faculté.

L'invention a ainsi pour objet un procédé de prise en compte des impulsions de localisation délivrées par une gama-caméra, tel que décrit dans la revendication. Ainsi,

— des scintillations produites par un rayonnement gamma dans un cristal scintillateur sont détectées et amplifiées par un réseau de tubes photomultiplicateurs pour constituer des contributions électriques de ces tubes,

— les contributions électriques sont successivement pondérées dans des matrices de résistances, transmises par des circuits de transmission si l'amplitude maximum de la scintillation appartient à une gamme prédéterminée, puis intégrées dans des intégrateurs pour produire des impulsions intégrées,

— les impulsions intégrées sont traitées pour produire lesdites impulsions de localisation, caractérisée en ce que

— la prise en compte est validée si l'énergie de la scintillation appartient à une bande d'énergie prédéterminée.

— la mesure de l'énergie de scintillation est réalisée par intégration de l'amplitude de la scintillation.

L'invention sera mieux comprise à la lecture de la description qui suit et à l'examen des figures qui l'accompagnent. Celles-ci ne sont données qu'à titre indicatif et nullement limitatif de l'invention. Les figures montrent :

— figure 1 : un dispositif pour la mise en oeuvre du procédé de l'invention ;

— figure 2a à 2g : des diagrammes temporels de signaux intervenant dans le procédé de l'invention ;

— figure 3 : la représentation statistique d'une fenêtre spectrométrique décalés utilisée pour constituer une plage de tolérance d'empilement selon l'invention.

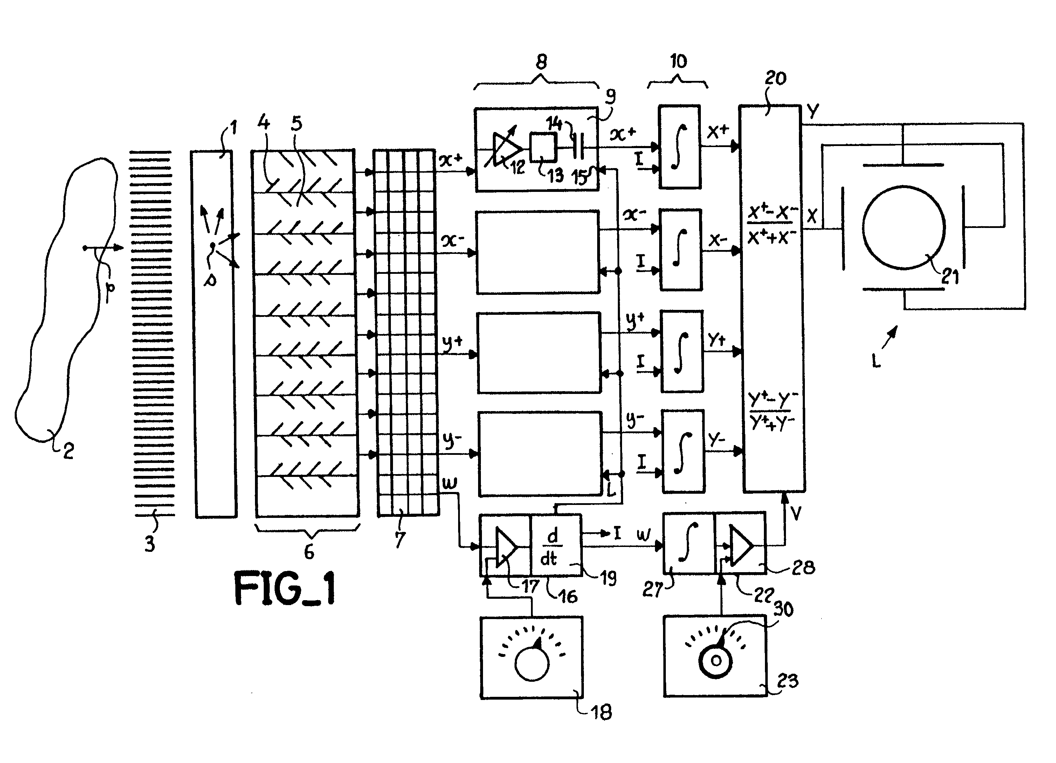

La figure 1 représente une gamma-caméra pour la mise en oeuvre du procédé selon l'invention. Cette gamma-caméra comporte essentiellement un scintillateur 1 pour recevoir des photons gamma "p" émis par un corps radioactif 2, éventuellement après qu'ils aient été focalisés par un collimateur 3. Des scintillations "s" sont émises sous la forme de photons lumineux qui viennent exciter les dynodes 4 des tubes photomultiplicateurs 5 d'un réseau 6 de tubes photomultiplicateurs disposé en face de ce scintillateur. Les scintillations sont alors transformées par chacun des tubes en des contributions électriques de tube. Chacune des contributions électriques de tube est introduite dans un jeu 7 de matrices de résistances qui élaborent des impulsions pondérées. Dans une gamma-caméra de type classique il y a essentiellement cinq matrices de résistances destinées à délivrer les impulsions pondérées respectivement $x^+$, $x^-$, $y^+$, $y^-$ et w. Les quatre premières sont représentatives de la localisation du lieu de la scintillation "s" sur le cristal 1, la cinquième "w" est représentative de l'amplitude de la scintillation. Cette dernière est souvent appelée l'énergie de la scintillation : elle n'est cette énergie que dans la mesure où ce signal est intégré dans le temps.

Un ensemble 8 de circuits de transmission tels que le circuit 9 assure la transmission des impulsions pondérées à un ensemble 10 d'intégrateurs tels que 11. Les circuits de transmission comportent essentiellement chacun un amplificateur 12 à gain variable, une ligne à retard 13, ainsi qu'un circuit 14 de restauration du potentiel de base. Ces particularités des circuits de transmission 9 correspondent à une solution préférée de réalisation. Ils sont étrangers à l'invention proprement dite. L'utilité des circuits de transmission 9 réside cependant dans le fait que ces circuits de transmission peuvent être commandés par une entrée de commande 15 qui reçoit un ordre logique "L" provenant d'un circuit 16 de détection de la présence d'une scintillation.

Le fonctionnement de cet ensemble est le suivant. Le circuit 16 qui reçoit le signal "w" représentatif de l'énergie de la scintillation délivre l'ordre "L" de validation du fonctionnement des circuits de transmission 9 lorsqu'un comparateur 17 à seuil détecte que le signal "w" dépasse un seuil prédéterminé, ce seuil est fixé par un dispositif d'affichage 18, et lorsqu'un circuit de différenciation 19 détecte, ultérieurement au franchissment du seuil, la présence d'une crête du signal représentatif de l'énergie. En définitive la ligne à retard 13 sert à autoriser la restauration du potentiel de base par le circuit 14, en réponse à l'ordre "L", avant que cette impulsion pondérée soit transmise à un intégrateur du jeu 10 des intégrateurs. Les impulsions pondérées et transmises sont alors intégrées dans les intégrateurs. Ceux-ci délivrent respectivement à leur sortie les impulsions intégrées de localisation $X^+$, $X^-$, $Y^+$ et $Y^-$. Un circuit de traitement 20 d'un type connu élabore alors les impulsions de localisation proprement dites. Celles-ci sont ensuite appliquées sur les plaques de déflexion d'un oscillographe cathodique 21 tandis que l'ordre logique "L" est appliqué à l'électrode de WEHNELT de cet oscillographe cathodique pour provoquer l'apparition d'un impact sur l'écran de cet oscillographe.

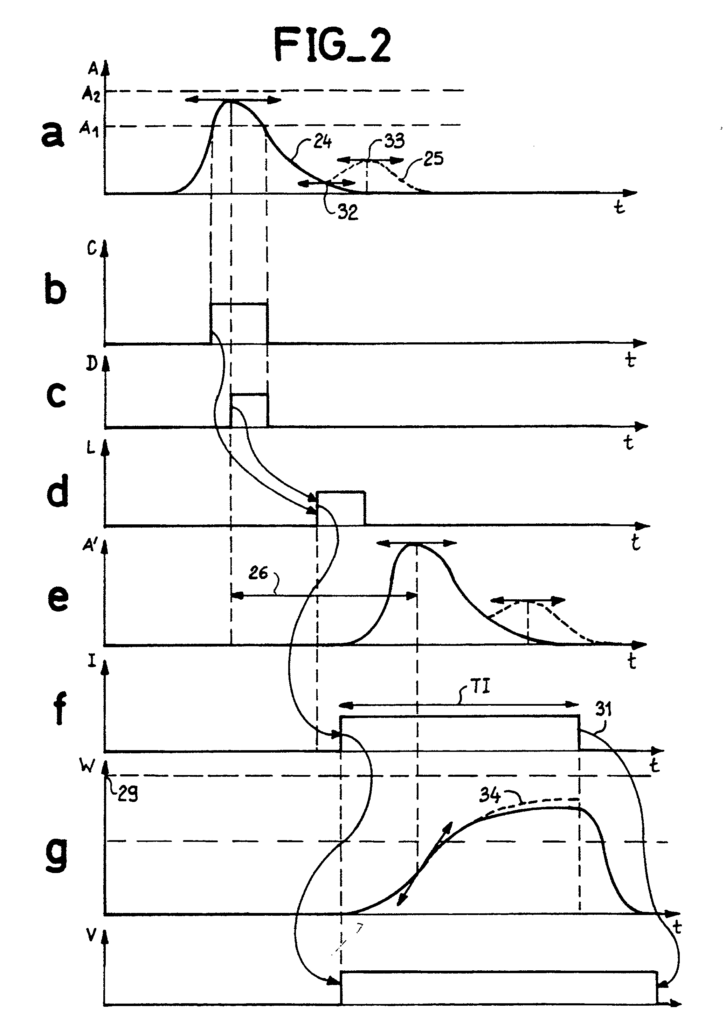

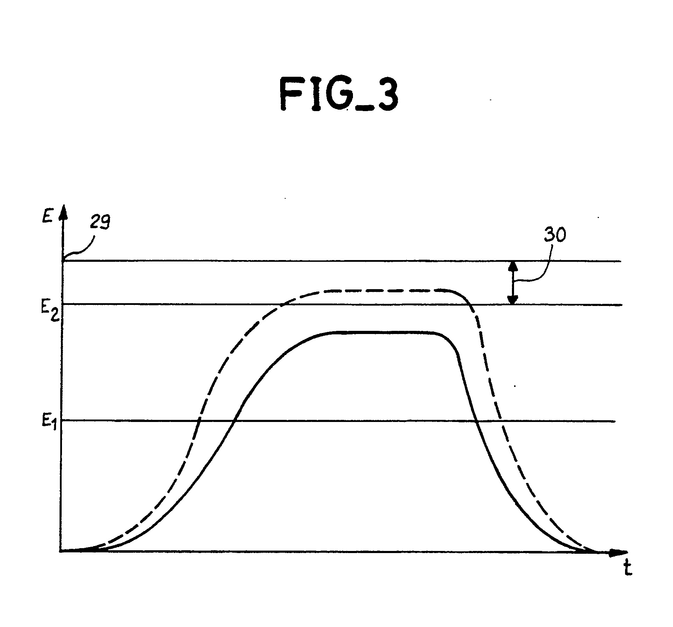

Ce qui caractérise l'invention est la mise en oeuvre d'un circuit 22 de mesure de l'énergie de la scintillation et de comparaison de cette énergie à une bande d'énergie prédéterminée. La bande d'énergie prédéterminée peut être indiquée par un dispositif d'affichage 23. Les figures 2a à 2h et la figure 3 permettent de mieux comprendre l'invention. La figure 2a représente le diagramme temporel de l'amplitude d'une scintillation 24 suivie d'un parasite 25 qui altère sa signification. La détermination que la scintillation appartient à une fenêtre spectrométrique E1-E2 (figure 3) est assimilée au fait que l'amplitude maximum du signal pondéré "w" représentatif de cette énergie appartient à une gamme prédéterminée A1-A2. Lorsque l'amplitude du signal "w" devient temporellement supérieure au seuil inférieur A1 de la gamme, le comparateur 17 émet un ordre C (figure 2b). Lorsqu'ultérieurement le signal "w" culmine dans la plage A1-A2 le différenciateur 18 émet un ordre D (figure 2c). Les ordres C et D sont alors utilisés dans un circuit logique contenu dans le circuit 16 pour produire un ordre L (figure 2d) de validation de la transmission des impulsions pondérées.

L'ordre L, légèrement retardé pour tenir compte de l'opération de restauration de potentiel de base, devient un ordre I (figure 2f) appliqué aux intégrateurs du jeu 10. Les intégrateurs tels que 11 intègrent alors les impulsions pondérées et transmises qui leur parviennent après un retard 26 imposé par la ligne à retard 13(figure 2e). De manière connue, la durée d'intégration TI des intégrateurs 11 est prédéterminée et est égale à un multiple de la durée de décroissance du signal de scintillation. Cette durée d'intégration est généralement égale au triple de cette constante de décroissance. Pendant toute la durée de cette intégration les intégrateurs associés aux impulsions transmises et représentatives de la localisation intègrent le signal qui leur parvient. De même un intégrateur 27 contenu dans le circuit 22 intègre le signal "w" et le transforme en un signal W vraiment représentatif, à l'issue de cette intégration, de l'énergie de la scintillation prise en compte.

Le signal W est alors introduit dans un comparateur 28 où il est comparé à un seuil 29 élaboré par l'afficheur 23 (figure 2g). Le seuil 29 est supérieur au seuil E2 de la bande d'énergie étudiée (figure 3) d'une valeur 30 indiquée par l'afficheur. Le comparateur 28 émet en sortie un ordre V présent par exemple sous la forme d'une impulsion passant à l'état actif au moment de l'application de l'ordre L. L'impulsion représentant l'ordre V est soit désactivée à la fin de l'intégration ou même après cette intégration (flèche 31, figure 2f-figure 2h), soit désactivée en fonction du dépassement par le signal d'énergie W du seuil 29. L'ordre V dure un peu plus longtemps que l'ordre I d'intégration pour autoriser, à l'issue de cette intégration, le traitement, dans le circuit de traitement des impulsions de localisation, des impulsions intégrées de localisation. Par contre, si le signal de l'énergie W a franchi le seuil 29, le comparateur 28 fournit un signal de désactivation de V avant la transmission des impulsions intégrées de localisation de telle manière que le traitement par le circuit 20 soit interdit.

Dans l'état de la technique la présence de la scintillation parasite 25 était détectée, avec les inconvénients que l'on a cité, par le passage à zéro du signal différencié résultant du début de manifestation (32) de ce parasite ou du maximum (33) de ce parasite (figure 2a). Dans l'invention le parasite 25 retentit en un décalage 34 du signal représentatif de l'énergie de la scintillation. Si ce décalage est faible la prise en compte est autorisée. Elle est interdite dans le cas contraire. On remarque que, de ce point de vue, l'invention apporte une possibilité de réglage de la tolérance d'empilement. Aucune possibilité de réglage n'est envisageable si on utilise une technique de détection du passage par zéro du signal différencié : le parasite 25 est pris en compte à coup sûr (pour invalider) quelle que soit son importance.

La version présentée est une version préférée. Cependant on peut réduire le nombre des matrices dans le jeu de matrices en employant à la place d'impulsion pondérée "w" la somme des impulsions pondérées $x^+$, $x^-$, $y^+$ et $y^-$. Dans la mesure où la somme de ces impulsions pondérées permet d'apprécier l'amplitude de la scintillation, la présence de la matrice dite énergie devient inutile.

## Revendications

1. Procédé de prise en compte (V) des impulsions de localisation (X, Y) délivrées par une gamma-caméra dans laquelle
— des scintillations (s) produites par un rayonnement gamma (p) dans un cristal scintillateur (1) sont détectées et amplifiées par un réseau (6) de tubes (5) photomultiplicateurs pour constituer des contributions électriques de ces tubes,
— les contributions électriques sont successivement pondérées ($x^+$, $x^-$, $y^+$, $y^-$, w) dans des matrices (7) de résistances, transmises par des circuits (9) de transmission si l'amplitude maximum de la scintillation (w) appartient à une gamme (A1, A2) prédéterminée, puis intégrées dans des intégrateurs (11) pour produire des impulsions intégrées ($X^+$, $X^-$, $Y^+$, $Y^-$),
— les impulsions intégrées sont traitées (20) pour produire lesdites impulsions de localisation, caractérisé en ce que
— on mesure l'énergie de la scintillation
— la prise en compte est validée si la mesure de cette énergie (W) de la scintillation appartient à une bande d'énergie prédéterminée (29) et en ce que l'énergie de la scintillation est mesurée par intégration (27) de l'amplitude de cette scintillation.

2. Procédé selon la revendication 1 caractérisé en ce que la durée d'intégration (TI) de l'amplitude de la scintillation est égale à la durée d'intégration des contributions électriques pondérées et transmises.

3. Procédé selon l'une quelconque des revendications précédentes caractérisé en ce que bande d'énergie (30) est réglable (23) en fonction d'une tolérance d'empilement prédéterminée des scintillations.

4. Procédé selon l'une quelconque des revendications 1 à 3 caractérisé en ce que la validation de la prise en compte comporte la comparaison (28) à un seuil (29) de la valeur de l'énergie.

5. Procédé selon l'une quelconque des revendications 1 à 4 caractérisé en ce que la mesure par intégration de l'amplitude de la scintillation est appréciée par la somme des impulsions de localisation.

6. Procédé selon l'une quelconque des revendications 1 à 5 caractérisé en ce que la transmission comporte l'application (13) d'un retard, la restauration (14) d'un potentiel de base, et une autorisation (L) de la transmission.

7. Procédé selon l'une quelconque des revendications 1 à 6 caractérisé en ce que l'autorisation de la transmission comporte une comparaison (17) à un seuil (18) associée à une détection de crête (19).

8. Procédé selon la revendication 7 caractérisé en ce que la détection de crête comporte une différenciation et une comparaison à zéro.

9. Dispositif de prise en compte des impulsions de localisation (X, Y) délivrées par une gamma-caméra qui comprend un cristal scintillateur (1) recevant un rayonnement gamme (p) et délivrant des scintillations (s), un réseau (6) de tubes photomultiplicateurs détectant et amplifiant les scintillations et délivrant des contributions électriques, des moyens de pondération (7) délivrant les contributions électriques pondérées (x⁺, x⁻, y⁺, y⁻, w), des circuits de transmission (9), des intégrateurs (11) délivrant des signaux intégrés (X⁺, X⁻, Y⁺, Y⁻), des moyens de traitement (20) délivrant des impulsions de localisation (X, Y) caractérisé en ce qu'il comprend des moyens de mesure (7, 16, 18) de l'énergie de scintillation (W) et des moyens de comparaison (22) aptes à délivrer un signal de validation (V) permettant de valider la prise en compte des impulsions de localisation lorsque la mesure de cette énergie appartient à une bande d'énergie prédéterminée et en ce que les moyens de mesure comportent un comparateur à seuil (17) suivi d'un différentiateur (19) et en ce que les moyens de comparaison (22) comportent un intégrateur (27) suivi d'un comparateur à seuil (28).

**Patentansprüche**

1. Verfahren zur Bestimmung (V) der Lokalisierungsimpulse (X, Y) von einer Gamma-Kamera, bei dem

Szintillationen (s), die von einer Gammastrahlung (p) in einem Szintillatorkristall (1) erzeugt werden, detektiert und von einem Netz (6) von Fotovervielfacherröhren (5) verstärkt werden, um elektrische Beiträge dieser Röhren zu bilden, die elektrischen Beiträge nacheinander in Widerstandsmatrizen (7) gewichtet werden (x⁺, x⁻, y⁺, y⁻, w), von Übertragungskreisen (9) übertragen werden, wem die maxiamale Amplitude der Szintillationen (w) in einem vorbestimmten Bereich (A1, A2) auftritt, dann Integratoren (11) integriert werden, um integrierte Impulse (X⁺, X⁻, Y⁺, Y⁻) zu erzeugen, die integrierten Impulse behandelt werden (20), um die Lokalisierungsimpulse zu erzeugen, dadurch gekennzeichnet, daß man die Szintillationsengerie mißt, die Bestimmung gültig ist, wem das Maß dieser Szintillationsenergie (W) in einem vorbestimmten Energieband (29) erscheint und daß die Szintillationsenergie durch Integration (27) der Amplitude dieser Szintillation gemessen wird.

2. Verfahren nach Anspruch 1, dadurch gekennzeichnet, daß die Integrationsdauer (Ti) der Szintillationsamplitude gleich der Integrationsdauer der gewichteten und übertragenen elektrischen Beiträge ist.

3. Verfahren nach einem der vorhergehenden Ansprüche, dadurch gekennzeichnet, daß das Energieband (30) in Abhängigkeit von einer vorbestimmten Packungstoleranz der Szintillationen regelbar ist (23).

4. Verfahren nach einem der Ansprüche 1 bis 3, dadurch gekennzeichnet, daß die Validierung der Bestimmung den Vergleich (28) mit einer Schwelle (29) des Energiewertes umfaßt.

5. Verfahren nach einem der Ansprüche 1 bis 4, dadurch gekennzeichnet, daß die Messung durch Amplitudenintegration durch die Summe der Lokalisierungsimpulse bewertet wird.

6. Verfahen nach einem der Ansprüche 1 bis 5, dadurch gekennzeichnet, daß die Übertragung die Anwendung (13) einer Verzögerung, die Wiedererstellung (14) eines Basispotentials und eine Ermächtigung (L) der Übertragung umfaßt.

7. Verfahren nach einem der Ansprüche 1 bis 6, dadurch gekennzeichnet, daß die Ermächtigung der Übertragung einen Vergleich (17) mit einem Schwellenwert (18) umfaßt, der einer Spitzendetektierung (19) zugeordnet ist.

8. Verfahren nach Anspruch 7, dadurch gekennzeichnet, daß die Spizendetektierung eine Differenzierung und einen Vergleich mit Null umfaßt.

9. Vorrichtung zur Bestimmung der Lokalisierungsimpulse (X, Y), die von einer Gammakamera geliefert werden, umfassend ein Szintillatorkristall (1), das eine Gammastrahlung (p) aufnimmt und Szintillationen (s) abgibt, ein Netz (6) von Fotovervielfacherröhren, die die Szintillationen detektieren und verstärken und elektrische Beiträge abgeben, Gewichtungseinrichtungen (7), die gewichtete elektrische Beiträge (x⁺, x⁻, y⁺, y⁻, w) abgeben, Übertragungskreise (9), Integratoren (11), die integrierte Signale (X⁺, X⁻, Y⁺, Y⁻) abgeben, Behandlungseinrichtungen (20), die Lokalisierungsimpulse (X, Y) abgeben, dadurch gekennzeichnet, daß sie Meßeinrichtungen (7, 16, 18) für die Szintillationsenergie (W) und Vergleichseinrichtungen (22) enthält, die dazu bestimmt sind, ein Validierungssignal (V) abzugeben, das es erlaubt, die Bestimmung der Lokalisierungsim-

pulse zu validieren, wenn der Meßwert dieser Energie in einem vorbestimmten Energieband auftritt, und daß die Meßeinrichtung einen Schwellenkomparator (17) enthält, dem ein Differenzierer (19) folgt und daß die Vergleichseinrichtungen (22) einen Integrator (27) enthalten, dem ein Schwellenkomparator (28) folgt.

## Claims

1. Process for taking into account (V) of locating pulses (X, Y) supplied by a gamma camera in which
   — scintillations (s) produced by a gamma radiation (p) in a scintillation crystal (1) are detected and amplified by a group (6) of photomultiplier tubes (5) for constituting electrical contributions of said tubes,
   — the electrical contributions are successively weighted ($x^+$, $x^-$, $y^+$, $y^-$, w) in resistor arrays (7), transmitted by transmission circuits (9) if the maximum amplitude of the scintillation (w) belongs to a predetermined range (A1, A2) and then integrated in integrators (11) for producing integrated pulses ($X^+$, $X^-$, $Y^+$, $Y^-$),
   — the integrated pulses are processed (20) for producing said locating pulses, in which
   — the energy of the scintillation is measured and the taking into account is validated if the measurement of said energy (W) of the scintillation belongs to a predetermined energy band (29) and in that the energy of the scintillation is measured by integration (27) of the amplitude of said scintillation.

2. Process according to claim 1, characterized in that the integration time (TI) of the amplitude of the scintillation is equal to the integration time of the weighted and transmitted electrical contributions.

3. Process according to any one of the preceding claims, characterized in that the energy band (30) is regulatable (23) as a function of a predetermined stacking tolerance of the scintillations.

4. Process according to any one of the claims 1 to 3, characterized in that the validation of the taking into account involves the comparison (28) with a threshold (29) of the energy value.

5. Process according to any one of the claims 1 to 4, characterized in that the measurement by integration of the amplitude of the scintillation is evaluated by the sum of the locating pulses.

6. Process according to any one of the claims 1 to 5, characterized in that the transmission involves the application (13) of a time delay, the restoration (14) of a basic potential and a transmmission authorization (L).

7. Process according to any one of the claims 1 to 6, characterized in that the transmission authorization involves a comparison (17) with a threshold (18) associated with a peak detection (19).

8. Process according to claim 7, characterized in that the peak detection involves a differentiation and a zero comparison.

9. Apparatus for taking into account locating pulses (X, Y) supplied by a gamma camera, which comprises a scintillation crystal (1) receiving a gamma radiation (p) and supplying scintillations (s), a group (6) of photomultiplier tubes detecting and amplifying the scintillations and supplying the electrical contributions weighting means (7) supplying weighted electrical contributions ($x^+$, $x^-$, $y^+$, $y^-$, w), transmission circuits (9), integrators (11) supplying integrated signals ($X^+$, $X^-$, $Y^+$, $Y^-$), processing means (20) supplying locating pulses (X, Y) characterized in that it comprises measuring means (7, 16, 18) of the scintillation energy (W) and comparison means (22) able to supply a validation signal (V) making it possible to validate the taking into account of the locating pulses when the measurement of said energy belongs to a predetermined energy band and in that the measuring means comprise a threshold comparator (17) followed by a differentiator (19) and in that the comparison means (22) comprise an integrator (27) folloowed by a threshold comparator (28).

**FIG_1**

# FIG_2

# FIG_3